(19) Europäisches Patentamt / European Patent Office / Office européen des brevets

(11) **EP 3 950 906 A1**

(12) **EUROPEAN PATENT APPLICATION**
published in accordance with Art. 153(4) EPC

(43) Date of publication:
**09.02.2022 Bulletin 2022/06**

(21) Application number: **20795788.7**

(22) Date of filing: **14.04.2020**

(51) International Patent Classification (IPC):
*C10N 20/00* (2006.01)    *C10N 40/30* (2006.01)
*C10M 105/42* (2006.01)

(52) Cooperative Patent Classification (CPC):
**C10M 105/42**

(86) International application number:
**PCT/JP2020/016390**

(87) International publication number:
**WO 2020/218082 (29.10.2020 Gazette 2020/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.04.2019 JP 2019083742**

(71) Applicant: **NOF Corporation
Shibuya-ku
Tokyo 150-6019 (JP)**

(72) Inventors:
• **UEDA, Seita
Amagasaki-shi Hyogo 660-0095 (JP)**
• **ODA, Kazuhiro
Amagasaki-shi Hyogo 660-0095 (JP)**
• **KAWAMOTO, Hideki
Amagasaki-shi Hyogo 660-0095 (JP)**

(74) Representative: **Mewburn Ellis LLP
Aurora Building
Counterslip
Bristol BS1 6BX (GB)**

(54) **ESTER FOR REFRIGERATOR OIL, AND WORKING FLUID COMPOSITION COMPRISING SAME**

(57)    (Object) To provide an ester for a refrigerator oil capable of excellent compatibility with a refrigerant in the case that it is used with a non-chlorine-based refrigerant.

(Solution) An ester for a refrigerator oil is composed of a ester reaction product of a component (A), component (B) and component (C). 0.02 to 0.4 mol of a constituent component derived from the component (B) and 3.2 to 3.96 mol of a constituent component derived from the component (C) are contained with respect to 1.0 mol of a constituent component derived from the component (A), and the formula (1) is satisfied.
(A) pentaerythritol
(B) a linear divalent carboxylic acid having 4 to 10 carbon atoms
(C) a monovalent carboxylic acid having 4 to 10 carbon atoms

$$\alpha \leqq \text{peak group (a)} \diagup \text{peak group (b)} \leqq \beta \cdots (1)$$

$$\alpha = -10 \times \text{ (a molar number of a constituent component derived from the component (B))} + 6.5 \cdots (2)$$

$$\beta = -10 \times \text{ (the molar number of the constituent component derived from the component (B))} + 7.5 \cdots (3)$$

(The peak group (a) is a total of an area of a peak observed in a retention time in 28 to 40 minutes obtained by a gas

**(Cont. next page)**

EP 3 950 906 A1

chromatography, and the peak group (b) is a total of an area of a peak observed in a retention time of 40 to 60 minutes obtained by the gas chromatography described above).

**Description**

Technical Field

The present invention relates to an ester for a refrigerator oil having excellent compatibility with a refrigerant. The invention further relates to an ester for a refrigerator oil used for a working fluid composition for a refrigerator oil containing R-32 refrigerant.

BACKGROUND ARTS

In refrigerating and air conditioning systems represented by air conditioning apparatuses such as a room air conditioner and packaged air conditioner, low-temperature apparatuses such as a refrigerating and cooling apparatus for household use, refrigerating apparatuses for industrial use and car air conditioners for a hybrid car, electronic car or the like, for considering the global environment and for reducing the greenhouse effect, refrigerants having high global warming potentials have been replaced with refrigerants having low global warming potentials. For example, R-32 refrigerant has been generally applied in air-conditioning apparatuses.
It is desirable that a refrigerant and refrigerator oil, which is a lubricating oil used in a compressor compressing the refrigerant, are compatible and do not separate in a wide range of temperature conditions. However, for example, R-32 refrigerant has the problem that it is hardly compatible with a refrigerator oil. For solving the problem, it has been developed an ester for a refrigerator oil which is compatible with R-32 refrigerant. According to patent document 1, it is disclosed that a tetra ester, incorporating a short-chain fatty acid and composed of pentaerythritol, 2-methyl propionic acid and 3,5,5-trimethyl hexanoic acid, exhibits excellent compatibility with R-32 refrigerant. The compatibility of the refrigerant and refrigerator oil may be the worst under the condition that the concentration of the refrigerator oil is several tens % with respect to the refrigerant, in many cases. According to patent document 1, it is thus disclosed that the compatibility is good under the condition that 10 mass% of the refrigerator oil is contained with respect to R-32 refrigerant.
Further, as a high pressure is needed in a cooling and air-conditioning system containing R-32 refrigerant, the discharge temperature in the compressor is high and consequently oil film of the refrigerator oil is thin, resulting in severe lubrication condition. It is thus necessary refrigerator oil having good lubricating property. For solving the problem, it is listed the methods of using an additive for improving the lubricating property and of applying an ester for the refrigerator oil having good lubricating property.
For example, as the ester for the refrigerator oil having good lubricating property, patent document 2 discloses a complex ester obtained by polycondensation of neopentyl glycol as a polyvalent alcohol, 1,4-butane diol and adipic acid as a polyvalent carboxylic acid. According to the complex ester having the oligomer structure, obtained by the polycondensation of the polyvalent alcohol and polyvalent carboxylic acid, the compatibility with the refrigerator oil is reduced while good lubricating property can be exhibited under the severe lubrication condition, by increasing the molecular weight.

(Prior technical documents)

(Patent documents)

(Patent document 1) WO 2012/026214 A1
(Patent document 2) WO 2014/017596 A1

SUMMARY OF THE INVENTION

(Object to be solved by the Invention)

However, according to the combination of R-32 refrigerant having low compatibility with refrigerator oils and the complex ester having the complex structure obtained by the polycondensation, the compatibility may rarely be deteriorated in the case that the concentration of the ester for refrigerator oil is low rather than in the case that the concentration of the ester for refrigerator oil is high usually providing lower compatibility. It is thus desired a complex ester having excellent compatibility even under the condition that the concentration of a refrigerator oil is low with respect to a refrigerant.
The present invention has been made considering the object provided by the prior arts described above and the object is, in the case that R-32 refrigerant is applied, to provide an ester for a refrigerator oil capable of achieving excellent compatibility with the refrigerant and a working fluid composition for a refrigerator containing the same.

(Solution for solving the object)

As the inventors have researched in depth for solving the problem described above, it is found that the ester, composed of a specific tetravalent alcohol, divalent carboxylic acid and monovalent carboxylic acid and satisfying a specific formula, exhibits excellent refrigerant compatibility with R-32 refrigerant. The present invention is thus made.
That is, the present invention is as follows.

(1) An ester for a refrigerator oil, said ester comprising an ester reaction product of components (A), (B) and (C) described below,

wherein the ester comprises a constituent component derived from said component (B) in a ratio of 0.02 to 0.4 mol and a constituent component derived from said component (C) in a ratio of 3.2 to 3.96 mol with respect to 1.0 mol of a constituent component derived from said component (A), and
wherein the ester satisfies the following formula (1).

(A) pentaerythritol
(B) a linear and divalent carboxylic acid having 4 to 10 carbon atoms
(C) a monovalent carboxylic acid having 4 to 10 carbon atoms

$$\alpha \;\leqq\; \text{peak group (a)} \diagup \text{peak group (b)} \;\leqq\; \beta \cdot \cdot \cdot (1)$$

$$\alpha = -10 \times \text{ (a molar number of said constituent component derived from said component (B))} +6.5 \cdot \cdot \cdot (2)$$

$$\beta = -10 \times \text{ (said molar ratio of said constituent component derived from said component (B))} +7.5 \cdot \cdot \cdot (3)$$

(In the formula (1),

said peak group (a) represents a total of an area of a peak observed in a retention time of 28 to 40 minutes in a gas chromatography, and
said peak group (b) represents a total of an area of a peak observed in a retention time of 40 to 60 minutes in said gas chromatography.)

(2) A working fluid composition for a refrigerator oil, the composition comprising:

R-32 refrigerant; and
the ester for the refrigerator oil of (1).

(Effect of the Invention)

According to the ester for the refrigerator oil of the present invention, it is possible to maintain good refrigerator compatibility in a wide range of mixed compositions, even in the case that R-32 refrigerant having a low dissolving power is applied.

BRIEF DESCRIPTION OF THE DRAWINGS

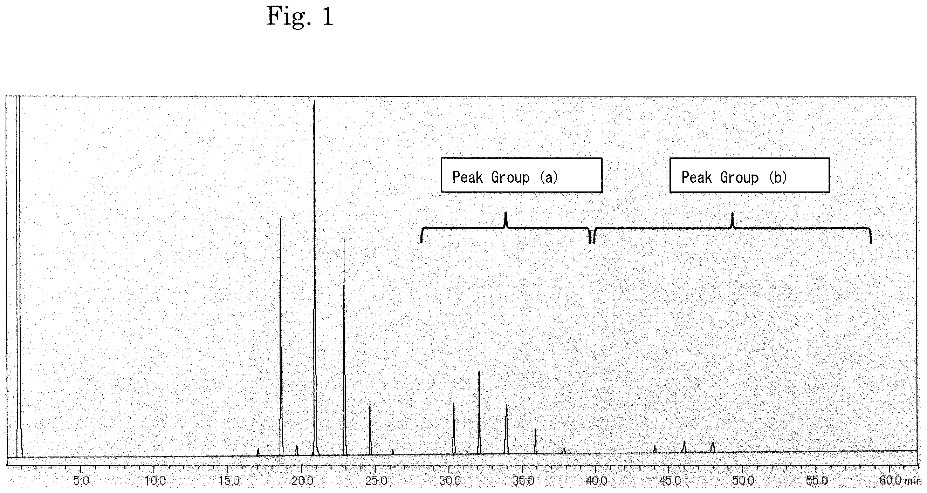

Fig. 1 is a chart indicating the results of analysis by a gas chromatography of an ester.

MODES FOR CARRYING OUT THE INVEBTION

Preferred embodiments of the present invention will be described further in detail below.

Further, each numerical range defined by a symbol "- " in the present specification is to include numerical values at both ends (upper limit and lower limit). For example, "2 - 5" means "2 or more and 5 or less".

The ester for the refrigerator oil of the present invention is obtained by mixing pentaerythritol (component (A)), a linear and divalent carboxylic acid having 4 to 10 carbon atoms (component (B)), and a monovalent carboxylic acid having 4 to 10 carbon atoms (component (C)) to perform the esterification.

Further, the terms of the component (A), component (B) and component (C) are general terms for the convenience, and a single kind of a compound may belong to each component or two or more kinds of compounds may be belong to each component. In the case that two or more kinds of compounds are contained in each component, the content of each component is defined as a total of contents of the two or more compounds belonging to the component.

As pentaerythritol of the component (A) used in the present invention, it may be used pentaerythritol available in the industry.

The component (B) is a linear and divalent carboxylic acid having 4 to 10 carbon atoms. In the case that the number of carbon atoms of the component (B) is less than 4, the compatibility with a refrigerant is deteriorated. The number of carbon atoms is 4 or more and preferably 6 or more. Further, in the case that the number of carbon atoms of the component (B) exceeds 10, the compatibility with a refrigerant is deteriorated, so that the number is 10 or less and preferably 8 or less. For example , the component (B) may be succinic acid, glutaric acid, adipic acid, pimelic acid, suberic acid, azelaic acid, sebacic acid or the like, and most preferably adipic acid.

The component (C) is the monovalent carboxylic acid having 4 to 10 carbon atoms, and may be each of linear and branched. The number of carbona atoms of the component (C) may preferably be 4 or more, or 8 or less. For example, the component (C) may be a linear fatty acid such as butanoic acid, pentanoic acid, caproic acid, heptanoic acid, octanoic acid, nonanoic acid, decanoic acid or the like or a branched fatty acid such as 2-methyl propionic acid, 2-methyl butanoic acid, 3-methyl butanoic acid, 3-methyl pentanoic acid, 4-methyl pentanoic acid, 2-ethylhexanoic acid, 3,5,5-trimethyl hexanoic acid or the like. Among these compounds, one kind may be used alone, or two or more kinds of compounds may be mixed and used.

As the component (C), it is particularly preferred to mix and use one or two or more kinds of (C1) a linear or branched fatty acid having 4 to 5 carbon atoms and (C2) one or two or more kinds of linear or branched fatty acid having 8 to 9 carbon atoms, in a molar ratio (C1/C2) in a range of 1/99 to 99/1. According to the present embodiment, it is more preferred to use (C1) 2-methyl propionic acid and (C2) 2-ethyl hexanoic acid in a molar ratio (C1/C2) in a range of 1/99 to 99/1.

The molar ratio ((C1)/(C2)) may more preferably be 40/60 to 90/10 and most preferably be 60/40 to 75/25.

The ester for the refrigerator oil of the present invention contains a constituent component derived from the component (B) in a ratio of 0.02 to 0.4 mol and a constituent component derived from the component (C) in a ratio of 3.2 to 3.96 mol, with respect to 1 mol of a constituent component derived from the component (A).

In the case that the content of the constituent component derived from the component (B) is less than 0.02 mol or more than 0.4 mol with respect to 1 mol of the constituent component derived from the component (A), it is impossible to maintain sufficiently high compatibility over a wide mixing ratio. The content of the constituent component derived from the component (B) is thus made 0.02 mol or more and 0.4 mol or less, is preferably 0.05 to 0.35 mol and more preferably 0.1 to 0.3 mol.

Further, the ratio of the constituent component derived from the component (C) is preferably 3.3 to 3.9 mol and more preferably 3.4 to 3.8 mol, with respect to 1 mol of the constituent component derived from the component (A).

The molar ratios of the respective constituent components described above are analyzed and calculated by gas chromatography.

That is, 0.1g of an ester is diluted in 5g of mixture of solvents of toluene and methanol (80wt%/20wt%) , into which 0.3g of 28% methanol solution of sodium methoxide (Wako Pure Chemical Corporation) is added and stood still at 60°C for 30 minutes to decompose the ester by methanolysis. The thus obtained solution of the decomposed ester is analyzed by gas chromatography to obtain ratios of areas of the thus obtained peaks of the component (A), component (B), component (C1) and component (C2), which can be converted to molar ratios for the calculation. Further, gas chromatography of each component alone is analyzed so that each component of the ester decomposition products can be identified.

The ester for the refrigerator oil of the present invention satisfies formula (1).

$$\alpha \;\leqq\; \text{peak group (a)} \diagup \text{peak group (b)} \;\leqq\; \beta \cdot \cdot \cdot (1)$$

$$\alpha = -10 \times \text{(molar number of the constituent component derived from the}$$

$$\text{component (B))} + 6.5 \cdots (2)$$

$$\beta = -10 \times \text{(molar number of the constituent component derived from the}$$

$$\text{component (B))} + 7.5 \cdots (3)$$

The peak group (a) and peak group (b) are to be calculated based on a chromatograph measured according to the following conditions.

Peak group (a): total of areas of peaks observed in a retention time in 28 to 40 minutes
Peak group (b): total of areas of peaks observed in a retention time of 40 to 60 minutes

That is, Fig. 1 is a chart showing the results of analysis by a gas chromatography of an ester. In Fig. 1, the peak group (a) is a total of the areas of the peaks observed in a retention time of 28 to 40 minutes, and the peak group (b) is a total of the areas of the peaks observed in a retention time of 40 to 60 minutes.

In the case that the value of the formula (1) is smaller than $\alpha$, the compatibility with the refrigerant is deteriorated. The value is thus made not smaller than $\alpha$, preferably $\alpha+0.1$ or larger and more preferably $\alpha+0.2$ or larger. Further, in the case that the value of the formula (1) exceeds 6, although the compatibility with the refrigerant is not deteriorated, the compatibility is not improved more and sufficient lubrication property may not be possibly obtained. The value is thus made $\beta$ or less, is preferably $\beta-0.1$ or less and more preferably $\beta-0.2$ or less.

The conditions for the gas chromatography for calculating the value of the formula (1) are made as follows. Measurement conditions :

Detecting apparatus : FID, temperature at supply inlet : 400°C,
temperature in a detector : 400°C
Column : "DB-1HT" supplied by Agilent Technologies Japan, Ltd. (length of 15m, inner diameter of 0.25mm)

Temperature at column oven: temperature of column is maintained at 60°C for 5 minutes from the start of the measurement, the temperature is elevated to 300°C at a rate of 10°C/min, elevated at 4°C/min to 400°C after reaching 300°C, and then maintained at 400°C for 16 minutes.

Carrier gas: helium (line speed : 55cm/sec)
Injection amount of sample : 1.0$\mu$L, Split ratio : 100
Preparation of sample : 0.1g of the ester for the refrigerator oil described above is diluted by 1.4g of toluene

When the ester is produced, all the components (A), (B) and (C) are charged into an appropriate reaction vessel and subjected to esterification reaction at ambient pressure and under nitrogen atmosphere. The esterification reaction is usually performed at 150 to 250°C, and preferably at 200 to 250°C, for efficiently removing water generated during the reaction. Further, the esterification reaction may be performed by using Bronsted acid catalyst or Lewis acid catalyst.

Further, a solvent may be used in the esterification reaction for efficiently removing water generated by the reaction into the out of the system. The solvent may be, for example, aromatic hydrocarbon solvent such as toluene, p-xylene or the like, hydrocarbon solvent such as cyclohexane, methyl cyclohexane or the like, or ketone solvent such as methyl isobutyl ketone, 3-pentanone or the like. Preferably, it may be a solvent having a boiling point of 100°C or higher and 200°C or lower. In the case that the solvent having a boiling point of lower than 100°C is used, the temperature in the reaction system is lowered and the progress of the esterification reaction becomes slow. In the case that the boiling point exceeds 200°C, the removal of the solvent from the reaction system is difficult. Further, on the viewpoint of satisfying the formula (1), it is more preferable to apply the solvent having a solubility parameter (SP value) in a range of 7.0 to 9.5 (cal/cm$^3$)$^{1/2}$. For example, p-xylene, methyl cyclohexane, 4-methyl-2-pentanone and the like are listed. Further, the solubility parameter (SP value) of the solvent can be calculated according to the formula of Fedor, and the details are described in "SP value, Basic • Application and Calculation methods" (authored by Hideki YAMAMOTO, published by JOHO KIKO Co. Ltd., 2006). The calculation is performed based on the description.

After the esterification reaction, unreacted raw materials and, in the case that the solvent is used, the solvent are removed by distillation under reduced pressure to obtain crude ester. Further, the crude ester is subjected to deoxidation by an alkali and then subjected to an operation such as adsorption treatment by an adsorption agent such as activated clay, acid clay or synthetic adsorption agent or steaming alone or in combination, so that the ester can be obtained.

The ester for a refrigerator oil of the present invention may be used alone as a base oil or may be mixed and used with other base oil. It may be further appropriately blended, depending on the objects, with known additives such as phenolic anti-oxidants, metal inactivating agents such as benzotriazoles, thiaziazoles, dithiocarbamates or the like, acid scavengers such as epoxy compounds, carbodiimide or the like, and phosphor-based extreme pressure agents.

As the ester for a refrigerator oil of the present invention has excellent compatibility with R-32 (difluoro ethane)refrigerant, it can be appropriately applied in a working fluid composition for a refrigerator containing R-32 refrigerant having low compatibility with the refrigerator oil.

Further, the refrigerant mixture containing R-32 may be used, and it may be listed mixed refrigerants such as R-407C (R-134a / R-125 / R-32 = 52 / 25 / 23 mass %), R-410R (R-125 / R-32 = 50 / 50 mass%), refrigerant mixture of R-1123 / R-32 = 40 / 60 mass%, or the like.

According to the working fluid composition for a refrigerator oil, the mass ratio of the ester for the refrigerator oil of the present invention and R-32 refrigerant (ester for the refrigerator oil / non-chlorine based freon refrigerant) is usually 1 / 99 to 90 / 10. In the case that the mass ratio of the refrigerant is in the range, the working fluid composition has appropriate viscosity, so that the lubricating property is excellent and the refrigerating efficiency is high, providing preferable embodiment.

EXAMPLES

Although the present invention will be described further in detail below referring to the following examples, the present invention is not limited to the examples.

(Inventive Example 1)

136.2g (1.00mol) of the component (A) (pentaerythritol), 38.0g (0.26mol) of the component (B) (adipic acid), 236.1g (2.68mol) of the component (C1) (2-methytl propionic acid), 151.4g (1.05mol) of the component (C2) (2-ethyl hexanoic acid), and 60g of 4-methyl-2-pentanone as reaction solvent were charged into a 1-liter 4-necked flask equipped with a thermometer, nitrogen supply tube, agitator, Dimroth condenser and oil-water separation tube having a volume of 30ml, and titanium isopropoxide was finally charged thereto in 0.2 molar equivalent with respect to the hydroxyl group of the charged alcohol.

The thus charged reaction liquid was heated under nitrogen air flow and subjected to the reaction at a temperature of 230°C until water generated by the reaction does not evaporate into the outside of the reaction system. Thereafter, the inside of the reaction vessel was cooled to 200°C and the pressure was reduced to 80 Torr to evaporate unreacted raw materials and the reaction solvent into the outside of the reaction system to obtain crude ester.

After the crude ester was cooled to 85°C, 1.5 equivalent of potassium hydroxide calculated based on the acid value was diluted with ion exchange water to produce 10% aqueous water, which was then added to the reaction liquid and agitated for 1 hour. After the agitation was terminated, the liquid was stood still for 30 minutes and water layer separated as the lower layer was removed. Then, 20 mass% of ion exchange water was added to the reaction liquid, agitated at 85°C for 10 minutes and stood still for 15 minutes. Then, the operation of removing the separated water layer was repeated until the pH of the water layer reached a value of 7 to 8. Thereafter, the dehydration was performed by agitation at 100°C and 30 Torr for 1 hour. Finally, 2 mass% of activated clay was added to the reaction liquid, which was agitated at 80°C and 30Torr for 1 hour, followed by the filtration by mean of a filter of 1 micron for removing the adsorption agent to obtain the compound of the inventive example 1.

(Inventive example 2)

136.2g (1.00mol) of the component (A) (pentaerythritol), 20.5g (0.14 mol) of the component (B) (adipic acid), 210.6g (2.39 mol) of the component (C1) (2-methyl propionic acid), 225.0g (1.56mol) of the component (C2) (2- ethyl hexanoic acid), and 60g of p-xylene as reaction solvent were charged into a 1-liter four-necked flask equipped with a thermometer, nitrogen supply tube, , agitator, Dimroth condenser and oil-water separation tube having a volume of 30ml. titanium isopropoxide was finally charged thereto in 0.2 mol equivalent with respect to hydroxyl group of the charged alcohol. The subsequent steps were performed according to the same procedure as that of the inventive example 1 to obtain a compound of the inventive example 2.

(Inventive Example 3)

136.2g (1.00mol) of the component (A) (pentaerythritol), 17.5g (0.12mol) of the component (B) (adipic acid), 195.1g (1.91mol) of the component (C1) (n-pentanoic acid), and 332.2g (2.10mol) of the component (C2) (3, 5, 5-trimethyl hexanoic acid) were charged into a 1-liter four-necked flask equipped with a thermometer, nitrogen supply tube, agitator, Dimroth condenser and oil-water separation tube having a volume of 30ml, and titanium isopropoxide was finally charged in 0.2 mol equivalent with respect to hydroxyl group of the charged alcohol. The subsequent steps were performed according to the same procedure as that of the inventive example 1, to obtain a compound of the inventive example 3.

(Inventive Example 4)

136.2g (1.00mol) of the component (A) (pentaerythritol), 58.5g (0.4mol) of the component (B) (adipic acid), 227.3g (2.58mol) of the component (C1) (2-methyl propionic acid), 131.2g (0.91mol) of the component (C2) (2-ethyl hexanoic acid) and 60g of 4-methyl-2-pentanone as reaction solvent were charged into a 1-liter 4-necked flask equipped with a thermometer, nitrogen supply tube, agitator, Dimroth condenser and oil-water separation tube having a volume of 30ml, and titanium isopropoxide was finally charged thereto in 0.2mol equivalent with respect to hydroxyl group of the charged alcohol. The subsequent steps were performed according to the same procedure as that of the inventive example 1, to obtain a compound of the inventive example 4.

(Inventive Example 5)

136.2g (1.00mol) of the component (A) (pentaerythritol), 58.5g (0.4 mol) of the component (B) (adipic acid), 313.6g (3.56 mol) of the component (C) (2-methyl propionic acid and 60g of p-xylene as reaction solvent were charged into a 1-liter 4-necked flask equipped with a thermometer, a thermometer, nitrogen supply tube, agitator, Dimroth condenser and oil-water separation tube having a volume of 30 ml, and titanium isopropoxide was finally charged thereto in 0.2 mol equivalent with respect to hydroxyl group of the charged alcohol. The subsequent steps were performed according to the same procedure as that of the inventive example 1, to obtain a compound of the inventive example 5.

(Comparative Example 1)

136.2g (1.00mol) of the component (A) (pentaerythritol), 35.1g (0.24 mol) of the component (B) (adipic acid), 254.6g (2.89 mol) of the component (C1) (2-methyl propionic acid) and 151.4g (1.05 mol) of the component (C2) (2-ethyl hexanoic acid ) were charged into a 1-liter 4-necked flask equipped with a thermometer, nitrogen supply tube, agitator, Dimroth condenser and a oil-water separation tube having a volume of 30ml, and titanium isopropoxide was finally charged thereto in 0.2 mol equivalent of hydroxyl group of the charged alcohol.
The thus charged reaction liquid was heated under nitrogen gas flow to 150°C, and the temperature was maintained over 48 hours. Thereafter, it was heated at 220°C and subjected to the reaction over 24 hours. Thereafter, it was filtered through a filter of 1 micron to obtain a compound of the comparative example 1.

(Comparative Example 2)

136.2g (1.00 mol) of the component (A) (pentaerythritol), 71.6g (0.49 mol) of the component (B) (adipic acid), 289.8g (3.29 mol) of the component (C) (2-methyl propionic acid) and 24.5g (0.17 mol) of the component (C2) (2-ethyl hexanoic acid) were charged into a 1-liter 4-necked flask equipped with a thermometer, nitrogen supply tube, agitator, Dimroth condenser and oil-water separation tube having a volume of 30ml, and titanium isopropoxide was finally charged thereto in 0.2 mol equivalent with respect to hydroxyl group of the charged alcohol. The subsequent steps were performed according to the same procedure as that of the inventive example 1, to obtain a compound of the comparative example 2.

(Calculation of value of formula (1))

The values of the peak group (a) and peak group (b) are calculated under the following conditions described below.
The measurement conditions are as follows.

Gas chromatography : "GC-2014" supplied by Shimadzu corporation
Detecting apparatus : FID

Temperature at supply inlet : 400°C
Temperature of the detecting apparatus: 400°C

Column : "DB-1HT" (length of 15m and inner diameter of 0.25mm) supplied by Agilent Technologies Japan, Ltd.

Temperature in column oven: The temperature at the column was maintained at 60°C for 5 minutes after the measurement was started, elevated to 300°C at a rate of 10°C/min, elevated to 400°C at a rate of 4°C/min after reaching 300°C, and then maintained at 400°C for 16 minutes.
Carrier gas: helium (line velocity : 55cm/sec)
Injection amount of sample : 1.0μL; Split ratio : 100
Preparation of sample : 0.1g of the ester for the refrigerator oil described above was diluted with 1.4g of toluene.

(Calculation of α and β)

They were calculated based on the molar ratios of the constituent components derived from the component (B) and on the formulas (2) and (3), respectively.

(Compatibility Test (1))

The samples of the inventive examples 1 to 3 and comparative examples 1 and 2 described above were prepared and subjected to the measurement of the two-layer separation temperature in a low temperature range under the condition that the mass ratio of the R-32 refrigerant and ester was 80:20, based on JIS K-2211. It was evaluated as "◎ " in the case that the two-layer separation temperature is -50°C or lower, "o" in the case that it is in a range of higher than -50°C and lower than -30°C, and "×" in the case it is -30°C or higher.

(Compatibility Test (2))

The respective samples were prepared and subjected to the measurement of the two-layer separation temperature in the low temperature range under the condition that the mass ratio of the R-32 refrigerant and ester was 95: 5, based on JIS K-2211. It was evaluated as "◎ " in the case that the two-layer separation temperature is -35°C or lower, "o" in the case that it is in a range of higher than -35°C and lower than -25°C, and "×" in the case it is -25°C or higher.
Table 1 and table 2 show the results of the formula (1) and compatibility tests of the inventive examples 1 to 5 and comparative examples 1 and 2, respectively.

Table 1

| | | Inventive Example 1 | Inventive Example 2 | Inventive Example 3 | Inventive Example 4 | Inventive Example 5 |
|---|---|---|---|---|---|---|
| Constituent components (molar ratio with respect to 1 mol of component A) | Component (A) | 1 | 1 | 1 | 1 | 1 |
| | Component (B) | 0. 25 | 0. 14 | 0. 12 | 0. 38 | 0. 38 |
| | Component (C) | 3. 5 | 3. 72 | 3. 76 | 3. 24 | 3. 24 |
| | Component (C1) | 2. 5 | 2. 23 | 1. 88 | 2. 43 | 3. 24 |
| | Component (C2) | 1 | 1. 49 | 1. 88 | 0. 81 | 0 |
| Molar ratio (C1) / (C2) | | 71/29 | 60/40 | 50/50 | 75/25 | 100/0 |
| Peak group (a) /Peak group (b) | | 4. 3 | 5. 9 | 6. 1 | 3. 6 | 3. 5 |
| α calculated based on formula (2) | | 4 | 5. 1 | 5. 3 | 2. 7 | 2. 5 |
| β calculated based on formula (3) | | 5 | 6. 1 | 6. 3 | 3. 7 | 3. 5 |
| Compatibility test | (1) | ◎ (-50°C or lower) | ◎ (-50°C or lower) | ◎ (-50°C or lower) | ◎ (-50°C or lower) | ∘ (-48°C) |
| | (2) | ◎ (-39°C) | ∘ (-35°C) | ∘ (-28°C) | ∘ (-27°C) | ∘ (-26°C) |

Table 2

| | | Comparative Example 1 | Comparative Example 2 |
|---|---|---|---|
| Constituent components (molar ratio with respect to 1 mol of component (A)) | Component (A) | 1 | 1 |
| | Component (B) | 0. 25 | 0. 5 |
| | Component (C) | 3. 5 | 3. 00 |
| | Component (C1) | 2. 5 | 2. 85 |
| | Component (C2) | 1 | 0. 15 |
| Molar ratio (C1)/(C2) | | 71/29 | 95/5 |
| Peak group (a)/Peak group (b) | | 3. 1 | 2. 7 |
| $\alpha$ calculated based on formula (2) | | 4 | 1. 5 |
| $\beta$ calculated based on formula (3) | | 5 | 2. 5 |
| Compatibility test | (1) | o (-40°C) | × (25°C or higher) |
| | (2) | × (-21°C) | × (25°C or higher) |

As shown in the inventive examples 1 to 5, according to the present invention, it is possible to obtain the ester for the refrigerator oil, which exhibits excellent refrigerant compatibility and can be appropriately applied for a compressor of a wide range of refrigerating and air conditioning apparatuses.

According to the comparative example 1 in which the value calculated from the formula (1) is out of the range, the compatibility with the refrigerant is inferior under the condition that the concentration of the oil with respect to the refrigerant is 5%.

Further, according to the comparative example 2, in which the value calculated from the formula (1) is out of the range and the ratio of the constituent component derived from the component (B) is out of the range, the compatibility with the refrigerant is inferior under both conditions that the concentrations of the oil with respect to the refrigerant are 20% and 5%, respectively.

## Claims

1. An ester for a refrigerator oil, said ester comprising an ester reaction product of components (A), (B) and (C) described below,

wherein said ester comprises a constituent component derived from said component (B) in a ratio of 0.02 to 0.4 mol and a constituent component derived from said component (C) in a ratio of 3.2 to 3.96 mol with respect to 1.0 mol of a constituent component derived from said component (A), and
wherein said ester satisfies the following formula (1).

(A) pentaerythritol
(B) a linear and divalent carboxylic acid having 4 to 10 carbon atoms
(C) a monovalent carboxylic acid having 4 to 10 carbon atoms

$$\alpha \leqq \text{peak group (a)} \diagup \text{peak group (b)} \leqq \beta \cdots (1)$$

$$\alpha = -10 \times \text{(a molar number of said constituent component derived from said component (B))} + 6.5 \cdots (2)$$

$$\beta = -10 \times \text{ (said molar number of said constituent component derived}$$

$$\text{from said component (B))} + 7.5 \cdots (3)$$

(In the formula (1),

said peak group (a) represents a total of an area of a peak observed in a retention time of 28 to 40 minutes in a gas chromatography, and
said peak group (b) represents a total of an area of a peak observed in a retention time of 40 to 60 minutes in said gas chromatography.)

2.  A working fluid composition for a refrigerator oil, said composition comprising:

R-32 refrigerant; and
the ester for the refrigerator oil of claim 1.

Fig. 1

## INTERNATIONAL SEARCH REPORT

| | International application No. |
|---|---|
| | PCT/JP2020/016390 |

A. CLASSIFICATION OF SUBJECT MATTER
C10N 20/00(2006.01)n; C10N 40/30(2006.01)n; C10M 105/42(2006.01)i
FI: C10M105/42; C10N20:00 Z; C10N40:30

According to International Patent Classification (IPC) or to both national classification and IPC

B. FIELDS SEARCHED

Minimum documentation searched (classification system followed by classification symbols)
C10N20/00; C10N40/30; C10M105/42

| Documentation searched other than minimum documentation to the extent that such documents are included in the fields searched | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2013-507483 A (CHEMTURA CORPORATION) 04.03.2013 (2013-03-04) example 1, paragraphs [0039], [0050], [0051] | 1-2 |
| X | WO 2013/008487 A1 (KH NEOCHEM CO., LTD.) 17.01.2013 (2013-01-17) comparative example 1, paragraphs [0051], [0060] | 1-2 |
| A | JP 6-25690 A (THE LUBRIZOL CORPORATION) 01.02.1994 (1994-02-01) entire text | 1-2 |
| A | JP 4-220496 A (ASAHI DENKA KOGYO KK.) 11.08.1992 (1992-08-11) entire text | 1-2 |
| A | JP 2015-96470 A (KH NEOCHEM CO., LTD.) 21.05.2015 (2015-05-21) entire text | 1-2 |

☐ Further documents are listed in the continuation of Box C.    ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 01 July 2020 (01.07.2020) | 14 July 2020 (14.07.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**
Information on patent family members

International application No.

PCT/JP2020/016390

| Patent Documents referred in the Report | Publication Date | Patent Family | Publication Date |
|---|---|---|---|
| JP 2013-507483 A | 04 Mar. 2013 | US 2011/0079749 A1 example 1, paragraphs [0046], [0057], [0058] WO 2011/043905 A1 EP 2486112 A1 CN 102712862 A | |
| WO 2013/008487 A1 | 17 Jan. 2013 | CN 103649040 A | |
| JP 6-25690 A | 01 Feb. 1994 | EP 568348 A1 entire text KR 10-1993-0021755 A CN 1077983 A | |
| JP 4-220496 A | 11 Aug. 1992 | (Family: none) | |
| JP 2015-96470 A | 21 May 2015 | WO 2013/125511 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012026214 A1

- WO 2014017596 A1

**Non-patent literature cited in the description**

- **HIDEKI YAMAMOTO.** SP value, Basic Application and Calculation methods. JOHO KIKO Co. Ltd, 2006